# EUROPEAN PATENT SPECIFICATION

(11) **EP 2 561 840 B1**
(45) Date of publication and mention of the grant of the patent: **07.05.2014**
(21) Application number: 11178485.6
(22) Date of filing: 23.08.2011
(51) Int. Cl.: A61F 5/00

(54) **Device for anchoring an endoluminal sleeve in the GI tract**
Vorrichtung zum Verankern einer endoluminalen Hülse im GI-Trakt
Dispositif pour ancrer un manchon endoluminal dans l'appareil gastro-intestinal

(43) Date of publication of application: 27.02.2013
(73) Proprietor: ETHICON ENDO-SURGERY, INC., Cincinnati, OH 45242-2839 (US)
(72) Inventor: Voegele, James W., Cincinnati, Ohio 45249 (US); Berger, José M., Cincinnati, Ohio 45246 (US); Aronhalt, Taylor, Loveland, Ohio 45140 (US); Harris, Jason, Mason, Ohio 45040 (US); Zeiner, Mark S., Mason, Ohio 45040 (US); Stokes, Michael J., Cincinnati, Ohio 45244 (US)
(74) Representative: Leihkauf, Steffen Falk

(56) References cited:
- WO-A1-2008/096362
- WO-A2-2004/087014
- WO-A2-2009/086119
- US-A1- 2003 153 905
- US-A1- 2004 172 142
- US-A1- 2005 125 020

## Description

### FIELD OF THE INVENTION

The present invention relates generally to medical apparatuses and more particularly to devices for positioning and anchoring a lining to a hollow body organ, such as a stomach, intestine or gastrointestinal tract.

### BACKGROUND OF THE INVENTION

In cases of severe obesity, patients may currently undergo several types of surgery either to tie off or staple portions of the large or small intestine or stomach, and/or to bypass portions of the same to reduce the amount of food desired by the patient, and the amount absorbed by the gastrointestinal tract. The procedures currently available include laparoscopic banding, where a device is used to "tie off" or constrict a portion of the stomach, vertical banded gastroplasty (VBG), or a more invasive surgical procedure known as a Roux-En-Y gastric bypass to effect permanent surgical reduction of the stomach's volume and subsequent bypass of the intestine.

Although the outcome of these stomach reduction surgeries leads to patient weight loss because patients are physically forced to eat less due to the reduced size of their stomach, several limitations exist due to the invasiveness of the procedures, including time, general anesthesia, heating of the incisions and other complications attendant to major surgery. In addition, these procedures are only available to severely obese patients (morbid obesity, Body Mass Index >=40) due to their complications, including the risk of death, leaving patients who are considered obese or moderately obese with few, if any, interventional options.

In addition to the above described gastrointestinal reduction surgery, endoluminal sleeves are known for partially or totally lining certain portions of the stomach and of the intestine with the aim to separate or bypass at least part of the food flow from the lined portions of the gastrointestinal tract. It has been observed that by creating a physical barrier between the ingested food and certain regions of the gastrointestinal wall by means of endoluminal sleeves, similar benefits for weight loss and improvement or resolution of type 2 diabetes may be achieved as with gastric bypass surgery. Physicians believe that by creating a physical barrier between the ingested food and selected regions of the gastrointestinal wall, it might be possible to purposefully influence the mechanism of hormonal signal activation originating from the intestine. It was observed that endoluminal sleeves in certain regions of the stomach and the duodenum contributed to improve glycemic control and to reduce or eliminate other co-morbidities of obesity. Moreover the lining of parts of the Gi-tract by means of endosleeves provide an alternative or an additional therapy to traditional therapies of type II diabetes and obesity. Endosleeves may be placed in a brief and less invasive procedure and address the patient's fear of surgery. Contrary to traditional gastric bypass surgery, the result of endoluminal sleeve surgery is reversible and the sleeve can be removed after achievement of the clinical result, but also in case of the occurrence of undesired side effects or clinical complications.

A typical duodenal sleeve device is described in U.S. Pat. No. 7,267,694 where the proximal end of a flexible, floppy sleeve of impermeable material defining a sleeve lumen is endoscopically deployed and anchored with the help of a barbed stent in the pylorus or in the superior section of the duodenum, the stent also ensuring that the proximal lumen opening of the sleeve remains open. Chyme from the stomach, enters the proximal lumen opening of the sleeve and passes through the sleeve lumen to the distal lumen opening. Digestive enzymes secreted in the duodenum pass through the duodenum on the outside of the sleeve. The enzymes and the chyme do not mix until the chyme exits from the distal lumen opening of the liner tube. In such a way, the efficiency of the process of digestion of the chyme is diminished, reducing the ability of the gastrointestinal tract to absorb calories from the food.

G.I. Dynamics, Inc., (Watertown, Mass., USA) produces the Endobarrier(R) device that is substantially a duodenal sleeve device configured so that the proximal end of the device is anchored inside the duodenal bulb with the help of a barbed anchoring stent that also keeps the proximal lumen opening open.

In US 2004/0148034 is taught a duodenal sleeve device attached to a funnel, the funnel configured for anchored to the gastric walls inside the gastric cavity in proximity to the lower esophageal sphincter. Food passing the lower esophageal sphincter is directed by the funnel into the proximal lumen opening of the duodenal sleeve device.

In U.S. Pat. No. 7,121,283 is taught a duodenal sleeve device attached to a large stent-like anchoring device that presses outwardly against the pyloric portion of the stomach, the pyloric sphincter and the duodenal bulb.

In known endosleeves, it has been observed that the sleeve devices tend to move inside the GI tract and migrate away from their original anchoring position.

A further important issue with endoluminal sleeves is the risk of failure of sealing of the lined lumen and, hence, the risk of an undesired leakage of the partially digested food flow in the interstice between the, lumen wall and the sleeve. Moreover, known endoluminal sleeve attachment devices and methods are not yet fully satisfying with regard to permitting normal biological events, including vomiting, to occur. US2004/172142A1 discloses a satiation device having the features of the preamble of claim 1.

Further fields of desirable improvements related with endoluminal sleeves are their removal from the patient without injuring the involved tissues, the rapidity of deployment and removal of the sleeve, and the repeatability of the sleeve placement.

Accordingly, there is a need for improved devices and procedures for anchoring and sealing an endoluminal, particularly a duodenal sleeve in the GI tract.

### SUMMARY OF THE INVENTION

The present invention provides for an endoluminal, particularly duodenal, sleeve device according to claim 1 for the transoral, or endoscopic, positioning and anchoring of an endoluminal sleeve device within a gastrointestinal tract, including, but not limited to, the pylorus, the esophagus, stomach, duodenum as well as other portions of or the entire length of the intestinal tract, etc., unless specified otherwise. In the case of the present invention, the surgeon or endoscopist may insert devices as described below through the patient's mouth, down the esophagus and into the stomach or intestine as appropriate. The procedure can be performed entirely from within the patient's stomach or other intestinal tract, and does not necessarily require any external incision. Alternatively, the surgeon may insert devices as described below laparoscopically into the stomach or intestine as appropriate.

According to an aspect of the invention, there is provided a duodenal sleeve device according to claim 1.

The sleeve device can be endoluminally inserted in a stomach with the anchoring portion being in the folded collapsed shape and, once arrived inside the stomach, the anchoring portion can be unfolded in the expanded tubular shape which prevents the proximal sleeve end from passing distally through the pylorus.

In accordance with an aspect of the invention, the anchoring portion is made of a shape memory material having three separate phases, wherein a third phase corresponds to a third shape memory state in which the anchoring portion is folded back in a collapsed shape adapted for withdrawal thereof from the GI tract.

In accordance with a further aspect of the invention, the anchoring portion is made of a shape memory material having three separate phases, wherein a third phase corresponds to a third shape memory state in which a stiffness of the anchoring portion is reduced with respect to the stiffness thereof in the second shape memory state and adapted for folding the anchoring portion and withdrawal thereof from the G I tract.

In accordance with a further aspect of the invention, the anchoring portion is made of a shape memory material having three separate phases, wherein a third phase corresponds to a third shape memory state in which a shape and/or stiffness of the anchoring portion is altered (e.g. increased or reduced) with respect to the shape and stiffness thereof in the second shape memory state and adapted for adjusting the anchoring portion to anatomy.

In accordance with aspects of the invention, the anchoring portion may be configured that a transition between the shape memory states of the anchoring portion 8 can be induced either by a temperature change or by exposure to an electric field, by exposure to a magnetic field or by light irradiation and combinations thereof.

In accordance with a further aspect of the invention, the anchoring portion comprises a combination of two different two phase-shape-memory polymers with different glass transition temperatures, thereby achieving the three separate phases.

In accordance with a yet further aspect of the invention, the anchoring portion may comprise an electrical conductor or resistor adapted respectively to generate an electromagnetic field or to generate heat and having terminals for connecting an endoscopic electrode. The fixed position and extension of the electrical conductor or resistor within the anchoring portion assures that the phase transition triggering electromagnetic field or temperature is achieved throughout the shape memory material.

In accordance with a further aspect of the invention, an external surface of the anchoring portion forms one or more cavities or holes into which tissue of the gastrointestinal wall can invaginate or ingrow for additional anchoring.

A method (which is not part of the claimed invention, but the description of which helps to understand the invention and to appreciate the advantages) can hence be performed for internally lining a target section of the gastrointestinal tract of a patient, comprising:
- providing a sleeve device with an anchoring portion in shape memory material having at least two separate phases;
- endoscopically introducing the sleeve device in the GI tract and positioning of the anchoring portion in a target anchoring location with the anchoring portion folded in a collapsed shape of a first shape memory state,
- after positioning of the anchoring portion in the target anchoring location, triggering phase transition of the shape memory material thereby unfolding the anchoring portion in an expanded tubular shape of a second shape memory state and pressing the anchoring portion against a surrounding GI lumen wall, e.g. gastric wall proximal to a pylorus.

The shape and/or stiffness of the unfolded expanded anchoring portion can be adjusted by triggering a further phase transition of the shape memory material from the second shape memory state to a third shape memory state.

The method may comprise invaginating surrounding tissue of the GI wall, e.g. gastric wall, in one or more cavities formed in the anchoring portion.

After completion of the therapy, the sleeve device can be removed from the patient by cutting the anchoring portion or, alternatively, by triggering again a phase transition so that the anchoring portion folds back to a collapsed shape, thereby detaching from the surrounding tissue.

These and other aspects and advantages of the present invention shall be made apparent from the accompanying drawings and the description thereof, which illustrate embodiments of the invention and, together with the general description of the invention given above, and the detailed description of the embodiments given below, serve to explain the principles of the present invention.

### BRIEF DESCRIPTION OF THE DRAWINGS

- Figure 1 illustrates a duodenal sleeve device in accordance with an embodiment, an anchoring portion of the sleeve device being in a collapsed folded shape;
- Figure 2 illustrates a duodenal sleeve device in accordance with an embodiment, an anchoring portion of the sleeve device being in an expanded unfolded shape;
- Figure 3 illustrates a duodenal sleeve device in accordance with a further embodiment, an anchoring portion of the sleeve device being in an expanded unfolded shape;
- Figure 4 illustrates a duodenal sleeve device in accordance with a yet further embodiment, an anchoring portion of the sleeve device being in an expanded unfolded shape;
- Figures 5, 6 and 7 illustrate duodenal sleeve devices and non-claimed methods for anchoring the duodenal sleeve devices within a GI tract in accordance with further aspects of the invention;
- Figure 8 illustrates a detail of the duodenal sleeve device in Figure 5;
- Figures 9, 10, 11 illustrate non-claimed methods and devices for anchoring an endoluminal sleeve within a GI tract by artificially inducing adhesions in a gastrointestinal wall in accordance with a yet further aspect of the invention,
- Figures 12, 13 illustrate duodenal sleeve devices and non-claimed methods for anchoring the duodenal sleeve devices within a GI tract by means of dissolvable barbs and fasteners in accordance with a further aspect of the invention;
- Figures 14 and 15 illustrate duodenal sleeve devices and methods for avoiding sleeve lumen blockage in accordance with a further aspect of the invention;

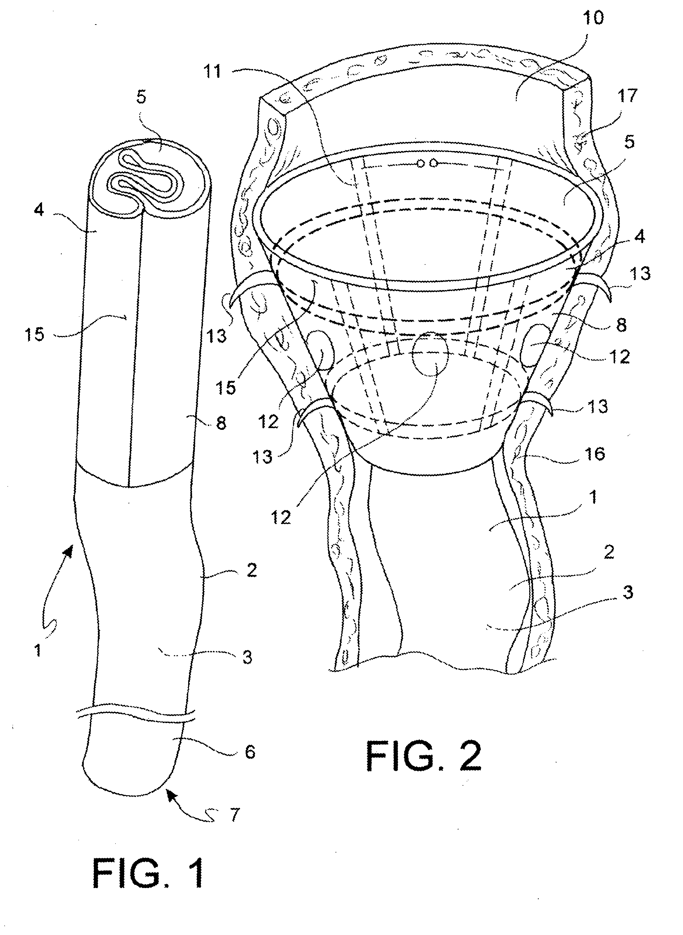

### Detailed Description of Device embodiments according to Figures 1 to 4

Referring to the drawings where like numerals denote like anatomical structures and components throughout the several views, an endoluminal sleeve device 1 for internally lining a section of the GI tract, particularly a section of duodenum distally from the pylorus, comprises a sleeve 2 configured for deployment inside a GI tract, particularly a duodenum of a human subject, the sleeve 2 having walls of a flexible material defining a sleeve lumen 3, a proximal end 4 defining a proximal lumen opening 5, and a distal end 6 defining a distal lumen opening 7. The device 1 further comprises an anchoring portion 8 forming the proximal sleeve end 4, the anchoring portion 8 being made of shape memory material having at least two separate phases corresponding to a first shape memory state and to a second shape memory state, wherein:
A) in the first shape memory state the anchoring portion 8 is folded in a collapsed shape,
B) in the second shape memory state, the anchoring portion 8 is unfolded in an expanded tubular shape adapted to shape interfere with a target lumen of the GI tract.

The sleeve device 1 can be endoluminally inserted in a stomach 10 with the anchoring portion 8 being in the folded collapsed shape and, once arrived inside the stomach 10, the anchoring portion can be unfolded in the expanded tubular shape which prevents the proximal sleeve end from passing distally through the pylorus.

In accordance with an embodiment of the invention, the anchoring portion 8 is made of a shape memory material having three separate phases, wherein a third phase corresponds to a third shape memory state in which the anchoring portion 8 is folded back in a collapsed shape adapted for withdrawal thereof from the GI tract.

In accordance with a further embodiment of the invention, the anchoring portion 8 is made of a shape memory material having three separate phases, wherein a third phase corresponds to a third shape memory state in which a stiffness of the anchoring portion 8 is reduced with respect to the stiffness thereof in the second shape memory state and adapted for folding the anchoring portion and withdrawal thereof from the GI tract.

In accordance with a further embodiment of the invention, the anchoring portion 8 is made of a shape memory material having three separate phases, wherein a third phase corresponds to a third shape memory state in which a shape and/or stiffness of the anchoring portion 8 is altered (e.g. increased or reduced) with respect to the shape and stiffness thereof in the second shape memory state and adapted for adjusting the anchoring portion to anatomy.

In accordance with embodiments of the invention, the anchoring portion is configured that a transition between the shape memory states of the anchoring portion 8 can be induced by a temperature change, by exposure to an electric field, by exposure to a magnetic field or by light irradiation and combinations thereof.

In accordance with an exemplary non limiting embodiment, the shape memory material of the anchoring portion 8 may be conveniently selected such that the anchoring portion 8 can be maintained in the first shape memory state (collapsed folded shape) at ambient temperature or by cooling below body temperature and switches to the second shape memory state (unfolded expanded shape) at a first activation temperature corresponding to a human body temperature and then to the third shape memory state (folded back or weakened reduced stiffness) at a second activation temperature higher than the first activation temperature. Alternatively, by light activation of the shape change of the anchoring portion 8, the temperature is not necessarily increased, but e.g. only the cross-linking density within a shape memory polymer may be influenced and purposefully increased or reduced, thereby changing the shape of the anchoring portion 8 and/or its stiffness. In accordance with exemplary non limiting embodiments, temperature activatable shape memory polymers may comprise sterenic polymer, liquid cristal polymer, veriflex polymer, polyurethanes with ionic or mesogenic components made by prepolymer method,block copolymers of polyethylene terephthalate (PET) and polyethyleneoxide (PEO), block copolymers containing polystyrene and poly(1,4-butadiene), ABA triblock copolymer made from poly(2-methyl-2-oxazoline) and polytetrahydrofuran, linear, amorphous polynorbornene (e.g. Norsorex® by CdF Chemie/Nippon Zeon) or organic-inorganic hybrid polymers consisting of polynorbornene units that are partially substituted by polyhedral oligosilsesquioxane (POSS), light activatable shape memory materials may comprise a polymer containing cinnamic groups, particularly cinnamic acid and cinnamylidene acetic acid, as photoresponsive switches and adapted to be fixed into the above said shape memory states by UV light illumination in different wavelength ranges (> 260 nm and < 260 nm), electric field activatable shape change materials may comprise composites of polymers charged with carbon nanotubes, short carbon fibers, carbon black particles or metallic Ni powder, remote magnetic field activatable chape change materials may comprise e.g. surface-modified super-paramagnetic nanoparticles in a polymer matrix, e.g. oligo (e-capolactone)dimethacrylate/butyl acrylate composite with magnetite nanoparticles and/or Nickel particles, metal shape change materials may comprise e.g. NiTinol.

In accordance with an embodiment, the anchoring portion 8 comprises a combination of two different double-shape-memory polymers with different glass transition temperatures, thereby achieving the three separate phases.

Depending from the specific therapeutic treatment plan, the anchoring portion 8 may comprise a shape memory polymer which may be non degradable to resist against the corrosive gastric juices and digestive fluids or, alternatively, the anchoring portion can be made of a biodegradable polymer which dissolves over time and which can be evacuated together with the stool.

In accordance with a further embodiment, the anchoring portion 8 may comprise an electrical heating resistance 9 with terminals for connecting an endoscopic electrode. The predetermined position and extension of the electrical resistance 9 within the anchoring portion 8 assures that the phase transition temperature is achieved throughout the shape memory material.

In accordance with a yet further embodiment, the anchoring portion 8 may comprise an electrical conductor 11 or conducting winding with terminals for connecting an endoscopic electrode and adapted to generate an electromagnetic field. Also in this embodiment, the fixed position and extension of the electrical conductor 11 within the anchoring portion 8 assures that the phase transition triggering electric field or magnetic field is achieved throughout the shape memory material.

In accordance with a yet further embodiment of the invention, the anchoring portion 8 may comprise an electrical conductor winding 14 adapted to induce an electric current in response to a magnetic field variation which may be generated by an extracorporeal magnetic field source. Also in this embodiment, the fixed position and extension of the electrical conductor winding 14 within the anchoring portion 8 assures that the phase transition triggering resistive heating or electrical field is achieved exactly in the planned regions of the shape memory material in the anchoring portion 8.

The anchoring portion 8 may further contain a radio-opaque substance, such as e.g. barium sulfate, to allow radiographic monitoring of the shape change and control that the planned expanded unfolded shape and position of the anchoring portion 8 is correctly achieved.

In accordance with a further embodiment (figure 2), the anchoring portion 8 has the shape of a truncated cone shaped tube in the unfolded expanded shape.

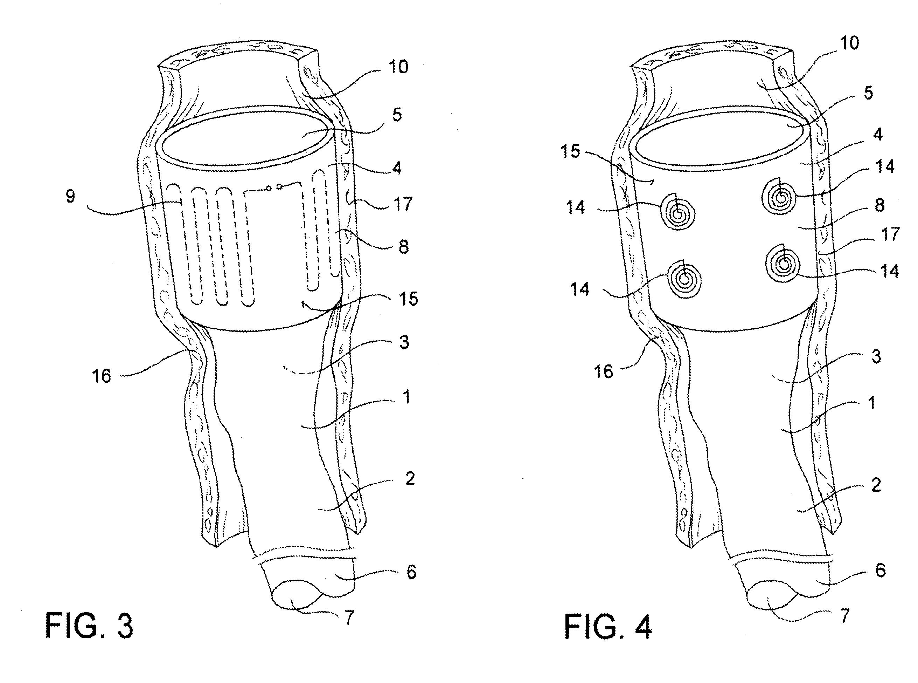

In accordance with a further embodiment (Figure 3), the anchoring portion 8 has the shape of a circular cylindrical tube in the unfolded expanded shape.

In accordance with a further embodiment, the anchoring portion 8, when in the unfolded expanded shape, may form one or more angled contour portions forming ridges or steps to improve engagement with the gastrointestinal wall.

In accordance with a further embodiment of the invention, an external surface 15 of the anchoring portion 8 forms one or more cavities 12 or holes into which tissue of the gastrointestinal wall can invaginate or ingrow for additional anchoring.

Additionally or alternatively, protrusions 13, e.g. barbs or bumps, can extend outward from the external surface 15 of the anchoring portion 8. These protrusions can be adapted to engage the surrounding tissue either by piercing the tissue or by deforming the tissue without piercing it.

Advantageously, the protrusions 13 are covered by folds when the anchoring portion 8 is in the collapsed folded shape of the first shape memory state, and the same protrusions 13 are exposed when the anchoring portion 8 is in the expanded unfolded shape of the second shape memory state. This facilitates endoluminal introduction of the device 1 and reliable anchoring thereof after introduction.

In accordance with a further embodiment, the sleeve 2 may be internally coated with a lubricant coating and/or with an antimicrobial coating to increase the flow of chyme through the sleeve lumen 3 and to avoid clogging and infection of the sleeve 2.

In accordance with a further embodiment of the invention a non-claimed method is provided for internally lining a target section of the gastrointestinal tract of a patient, comprising:
- providing a sleeve device 1 with an anchoring portion 8 in shape memory material having at least two separate phases;
- endoscopically introducing the sleeve device 1 in the GI tract and positioning of the anchoring portion 8 in a target anchoring location with the anchoring portion folded in a collapsed shape of a first shape memory state,
- after positioning of the anchoring portion 8 in the target anchoring location, triggering phase transition of the shape memory material thereby unfolding the anchoring portion 8 in an expanded tubular shape of a second shape memory state and pressing the anchoring portion 8 against a surrounding GI lumen wall, e.g. gastric wall 17 proximal to a pylorus 16.

In accordance with embodiments, the phase transition may be triggered by exposure of the anchoring portion 8 to the body temperature, by injecting a fluid (e.g. heated water, saline solution, air, CO₂) in the target anchoring location, the fluid having a temperature equal to a phase transition temperature of the shape memory material.

Alternatively, the phase transition may be triggered by introducing an endoscopic electrode to the anchoring portion 8 and either:
- heating the anchoring portion 8 directly by means of the endoscopic electrode;
- or attaching the endoscopic electrode to a contact terminal of an electric resistor 9 integrated in the anchoring portion 8 and heating the anchoring portion 8 by energizing the electric resistor 9;
- or attaching the endoscopic electrode to a contact terminal of an electric conductor 11 integrated in the anchoring portion 8 and inducing an electromagnetic field at the anchoring portion 8 by energizing the electric conductor 11.

In accordance with a yet further embodiment, the phase transition may be triggered by transcutaneous magnetic field generation by means of an extracorporeal magnetic field generator.

After unfolding the anchoring portion 8 in the expanded tubular shape of the second shape memory state, the shape and position of the anchoring portion 8 is checked by radiography.

In accordance with a further embodiment of the invention, the shape and/or stiffness of the unfolded expanded anchoring portion 8 can be adjusted by triggering a further phase transition of the shape memory material from the second shape memory state to a third shape memory state.

The further phase transition from the second shape memory state to the third shape memory state may be triggered by the same methods and devices described for the phase transition from the first shape memory state to the second shape memory state.

In accordance with a further embodiment, the non-claimed method may comprise invaginating surrounding tissue of the GI wall, e.g. gastric wall 17, in one or more cavities 12 formed in the anchoring portion 8.

After completion of the therapy, the sleeve device 1 can be removed from the patient by cutting the anchoring portion 8 or, alternatively, by triggering again a phase transition so that the anchoring portion 8 folds back to a collapsed shape, thereby detaching from the surrounding tissue.

In accordance with an exemplary embodiment, removal of the sleeve device 1 can be accomplished by a combination of triggering a phase transition which folds the anchoring portion 8 to a collapsed shape and cutting away tissue portions attached to the anchoring portion 8, such as e.g. invaginated tissue or ingrown tissue of a gastric wall 10 or of a duodenal wall.

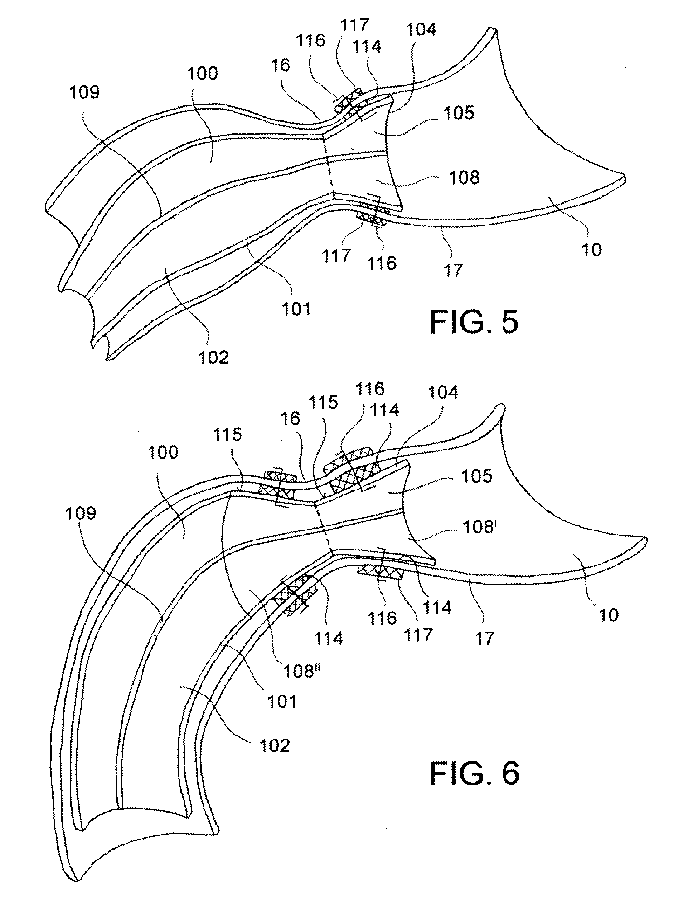

### Detailed Description of Device embodiments according to Figures 5 to 8

In accordance with yet further aspects of the present invention (Figures 5 through 8), there is provided an endoluminal sleeve device 100 for internally lining a section of the GI tract, the sleeve device 100 comprising a sleeve 102 configured for deployment inside a GI tract, particularly a duodenum of a human subject, the sleeve 102 having walls of a flexible material defining a sleeve lumen 103, a proximal end 104 defining a proximal lumen opening 105, and a distal end 106 defining a distal lumen opening 107.

The sleeve 102 may form one or more longitudinal stiffening ribs 109 to aid insertion in the GI tract and shape holding.

In accordance with an embodiment, a wall 101 of sleeve 102 comprises one or two initially flat sheets sealed and, possibly folded, together along overlapping longitudinal edges 110 of the sheets.

The thus obtained tubular sleeve wall 101 can be inserted over a heating mandrel and heat treated and shaped to obtain a tubular open shape, preferably with a funnel shaped or cone shaped anchoring portion 108 at the proximal end 104. The heat treatment of the sealed and folded overlapping longitudinal edges 110 creates a longitudinal seam of increased thickness which forms the longitudinal stiffening rib 109.

Alternatively, the sleeve 102 may be fabricated by injection molding, extrusion, blow molding, blow extrusion, i.e. extrusion and subsequent blow expansion or combinations of these methods.

The funnel shaped anchoring portion 108 of the sleeve wall 101 may be adapted to stretch to a large diameter in order to follow the antrum contour when it is distended, and to wrinkle down or retract to a smaller diameter in order to follow the antrum contour during antral contraction. This allows normal gastric peristalsis to occur and prevents undesired interference of the sleeve device 100 with the squeezing function of the antrum.

In accordance with an exemplary embodiment (Figure 5), the funnel shaped anchoring portion 108 has a non-symmetric cone shape suitable to adapt to both the gastric wall regions at the lesser curvature 111 and at the greater curvature 112.

In accordance with a further embodiment (Figure 6), the anchoring portion 108 forms a proximal funnel shaped portion 108' and a distal funnel shaped portion 108" connected to each other to form an hourglass shaped double cone intended and adapted to be inserted within the pyloric sphincter. This allows to anchor the sleeve device 1 in two positions proximally and distally to the pylorus 16. Also in this embodiment the proximal funnel shaped portion 108' and/or the distal funnel shaped portion 108" may have non symmetric cone or funnel shapes in order to better adapt to the shape of the pyloric sphincter, the antrum and the proximal duodenum.

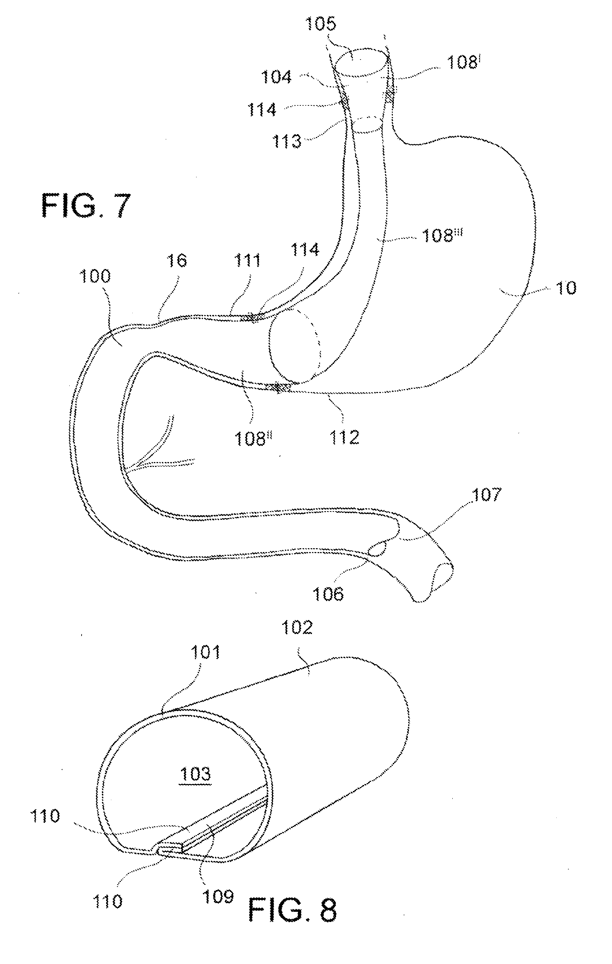

In accordance with a yet further embodiment (Figure 7), the anchoring portion 108 forms a proximal funnel shaped portion 108' and a distal funnel shaped portion 108" connected by a flexible tubular intermediate portion 108"', in which both the proximal and distal funnel shaped portions 108', 108" are tapered distally and in which the proximal funnel shaped portion 108' is intended and adapted to be anchored in the esophageal gastric junction (E-G junction) 113 and the distal funnel shaped portion 108" is intended and adapted to be anchored in the antrum or pylorus 16. This allows to anchor the sleeve device 1 in two positions at the entrance and exit of the stomach and to bypass the entire stomach 10. Thanks to the flexibility of the intermediate portion 108"', the nutrients entering the sleeve can be mechanically manipulated by the gastric wall motion, without however getting into contact with the gastric wall and with the gastric hormones, enzymes, endocrines, peptides etc. It is thus possible to alter the physiological signaling mechanisms triggered by such contact between food and the gastric environment.

For the purpose of a reliable anchoring and sealing of the anchoring portion to the gastrointestinal wall, a surgical mesh 114 or felt may be bonded to an external surface 115 of the anchoring portion 108, 108', 108" intended to face the GI wall. The surgical mesh 114 or felt extends preferably circumferentially all around the anchoring portion 108, 108', 108" and provides a reinforcement for an attachment suture 116 which can be used to hold the sleeve device 100 in place. Moreover, the surgical mesh 114 or felt is adapted to create an additional barrier against food passage between the sleeve wall and the surrounding tissue.

In accordance with a non-claimed anchoring method, an additional surgical mesh or felt ring 117 may be placed from outside the GI tract around the gastrointestinal wall, e.g. against the stomach serosa, at the position of the anchoring portion 108, 108', 108" and the suture 116 may be stitched through the additional felt ring 117, the gastrointestinal wall and the anchoring portion 108, 108', 108". In this manner, the additional felt ring 117 acts as a buttress for the attachment 116.

In accordance with embodiments, the attachment suture 116 itself may be performed by using individual suture points or a running stitch pattern, multiple pre-attached sutures (which may be pre-attached to the anchoring portion 108, 108', 108"), or sutures or pre-attached sutures in combination with an adhesive gluing (e.g. a layer of adhesive provided at the surgical mesh 114) for temporary fastening and sealing of the anchoring portion to the gastrointestinal wall. Advantageously, the temporary gluing can be used to assure and maintain a correct position of the anchoring portion 108, 108', 108" within a target anchoring location of the GI tract before and during the application of the attachment suture 116.

In accordance with a further embodiment, the sleeve wall 101 distally to the anchoring portion 108 may have a substantially straight cylindrical shape and a sleeve diameter which is selected to be equal or greater than the distended duodenum diameter of the patient.

In accordance with a yet further embodiment, the distal end 106 of sleeve 102 may be tapered distally and the distal sleeve opening 107 may define a narrowing which acts as a valve adapted to prevent an undesired retro-flow of chyme into the sleeve.

In accordance with a further embodiment, the sleeve wall 101 may comprise an internal and/or external low frictional coating adapted to facilitate and accelerate the passage of chyme or gastric contents through the sleeve 102.

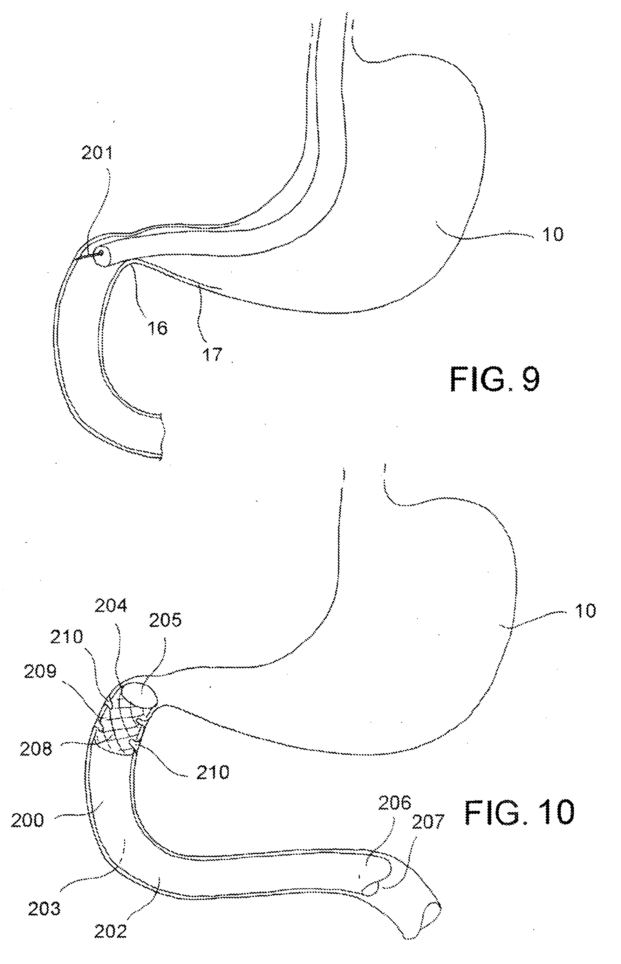

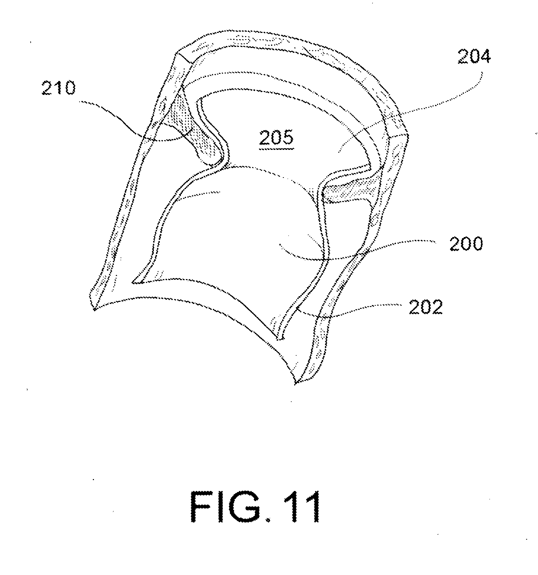

### Detailed Description of Device embodiments according to Figures 9 to 11

In accordance with a yet further aspect of the present invention (Figures 9 through 11), there is provided a non-claimed method and system for anchoring an endoluminal sleeve device 200 within a GI tract of a patient. The sleeve device 200 comprises a sleeve 202 configured for deployment inside a GI tract, particularly a duodenum of a human subject, the sleeve 202 having walls of a flexible material defining a sleeve lumen 203, a proximal end 204 defining a proximal lumen opening 205, and a distal end 206 defining a distal lumen opening 207. The sleeve device 200 may be generally configured as the previously described sleeve devices 1, 100.

The sleeve device 200 further comprises a perforated tissue anchoring structure 208, preferably formed at the proximal end 204 of the sleeve 202, and adapted to receive a permanent cell ingrowth of surrounding tissue of the GI wall, wherein the anchoring structure 208 is coated with a tissue ingrowth scaffold or promoter 209 adapted to induce and promote a tissue ingrowth into the perforations of the anchoring structure 208.

In accordance with embodiments, the tissue anchoring structure 208 may comprise a perforated fabric or a wire mesh, e.g. a Nitinol expandable stent, which may be formed circumferentially around the wall of the sleeve 202 in the positions in which anchoring of the sleeve 202 to the GI wall is planned.

In accordance with a further embodiment, the wall of the sleeve 202 itself may be perforated and coated with the tissue ingrowth scaffold or promoter 209 in order to form the anchoring structure 208.

In accordance with exemplary non limiting embodiments, the tissue ingrowth scaffold or promoter 209 may comprise:
- Keratin which is an endogenous growth media used by the human body to construct bodily tissue, and/or
- Collagen which has a similar mechanism of action as Keratin, and/or
- cellular poly-caprolactone.

The ingrowth scaffold or promoter 209 helps to initiate and direct the tissue ingrowth into the perforations of the anchoring structure 208 and provides a temporary scaffold for the ingrowing cells of the bodily tissue and for its vascularization, while the permanent anchoring occurs between the tissue and the anchoring structure 208 after the ingrowth scaffold 209 is eventually absorbed by the body.

The ingrowth of tissue into the perforations of the anchoring structure 208 may be further promoted by artificially inducing adhesions 210 (i.e. abnormal protrusions or bands of scar tissue) in the GI wall at the target anchoring location or locations. For this purpose, an adhesion agent adapted to artificially induce adhesions in bodily tissue, e.g. collagen, Keratin or tissue repair cells such as macrophages, fibroblasts and blood vessel cells, can be injected in the submucosa to form e.g. a permanent fibrous adhesion.

The injection of the adhesion agent can be accomplished by means of an endoscopic injection device 201 adapted and operable to inject the adhesion agent in the submucosa of the GI wall at a target anchoring location for the sleeve device 200.

Alternatively or additionally, the formation of adhesions may be induced by electrocauterization or chemical cauterization.

In accordance with a further embodiment, the adhesions of the GI wall may be induced to purposefully modify the geometry of the target anchoring location within the GI tract, in order to create a physical barrier, e.g. an internally protruding scar tissue flange, against displacement of the sleeve device 200.

Advantageously, the step of deploying and anchoring the sleeve device 200 to the adhesions 210 in the target anchoring location is performed after the step of inducing the adhesions and after an additional waiting phase which allows the induced adhesions 210 to grow and solidify. This prevents the adhesions 210 from being damaged by applying the anchoring forces during a too early stage of their development.

In accordance with embodiments, the additional waiting phase may last from 8 hours to 14 days, preferably from 2 days to 3 days.

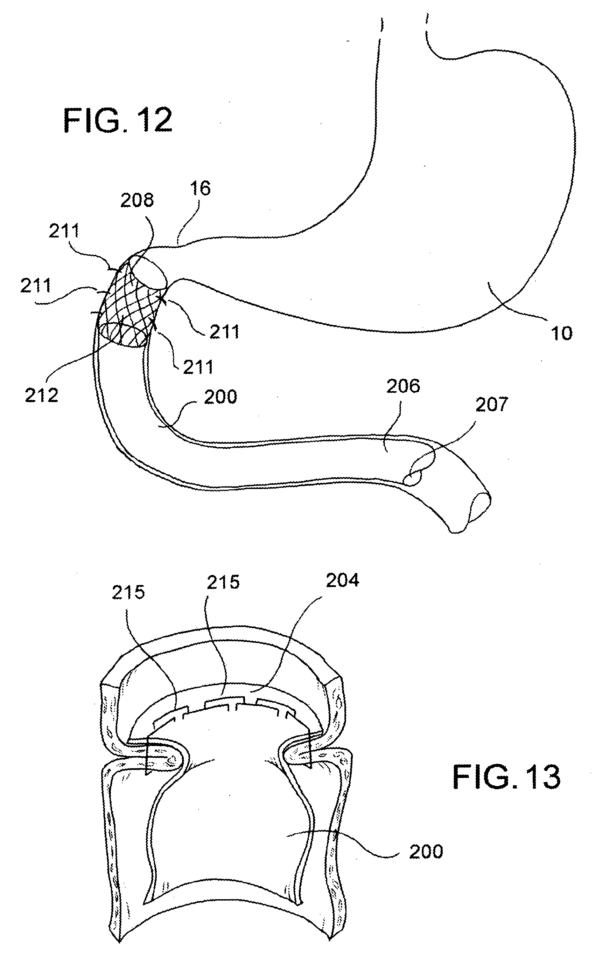

### Detailed Description of Device embodiments according to Figures 12 to 15

In accordance with a further embodiment of the invention (Figures 12) which can be advantageously applied to the previously described sleeve devices 1, 100, 200, the sleeve may comprise an anchoring portion (preferably at a proximal sleeve end) with barbs 211 which are adapted to pierce the surrounding tissue and which time dependently disintegrate, thereby creating a temporary anchoring which may be required during a period of tissue ingrowth into the sleeve or during a short term sleeve therapy without any need for endoscopically removing the sleeve device after the therapy is terminated.

In accordance with an embodiment, the barbs 211 may be bioabsorbable or biofragmentable such that they dissolve over time when exposed to the bodily fluids within the GI tract of a patient. Advantageously, the barbs 211 may be in 440C stainless steel which has shown to be safe and to definitely dissolve in the acid environment of the GI tract.

The barbs 211 may be supported by a Nitinol expandable stent 212 at an anchoring portion of the sleeve which may form an anchoring structure for the ingrowth of tissue and which can be deployed e.g. by means of a balloon dilator (not shown). The time dependently dissolvable barbs 211 will hold the sleeve device within the GI tract, e.g. within the duodenum, and maintain the patient on a restricted diet for several weeks during which the proximal sleeve end adheres itself into the gastrointestinal wall, thereby creating a permanent anchoring. After a certain amount of time, generally several weeks, which can be purposefully adjusted by adequately dimensioning and/or coating the barbs 211, the barbs will start to dissolve away leaving a safe long term sleeve implanted in the GI tract. Alternatively, by preventing a tissue ingrowth, after or before the barbs 211 are dissolved, the sleeve itself which may also be dissolvable is naturally evacuated from the body.

In accordance with a further embodiment, instead of or additionally to the time-dependently dissolvable barbs 211, the sleeve may be temporarily anchored by means of a bioabsorbable or biofragmentable circular clip or staple line 215 (Figure 13).

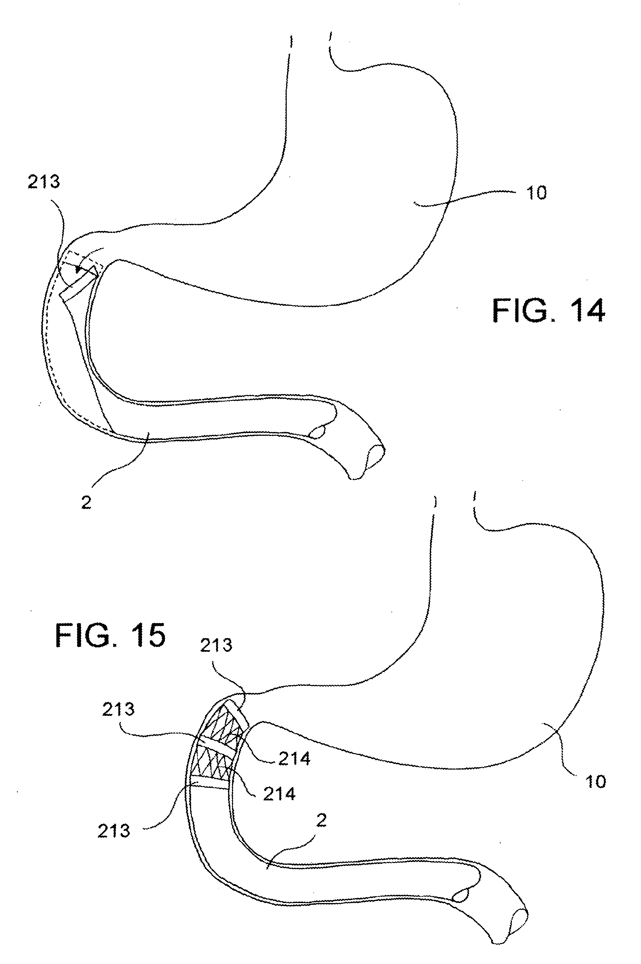

With reference to Figures 14 and 15, the sleeve 2, 102, 202 may be provided with rigid or semi-rigid rings 213 in order to maintain an open tubular sleeve shape. However, as has been indicated in Figure 14, the rings 213 may turn over and cause partial or total blockage of the sleeve lumen. This undesired slinkery effect of the rings 213 may be avoided by connecting a plurality of rings 213 to each other by means of rigid or elastic parallel or crossed connecting bars 214 or meshes, thereby creating an elastic or rigid tubular frame section which prevents the individual ring 213 from turning over.

The sleeve 2, 102, 202 itself is sufficiently flexible to follow the curvature of the duodenum. Further, in some embodiments the walls of the sleeve are sufficiently flexible and/or collapsible to allow duodenal peristalsis to drive chyme through the lumen of the sleeve. Sufficient collapsibility of the walls of the sleeve prevents continuous intimate contact of the outer surface of the sleeve with the duodenal mucosa, avoiding damage to the duodenal mucosa and allowing digestive secretions not collected into the sleeve lumen to pass through the duodenal lumen outside the sleeve lumen.

In some embodiments, at least a portion of the wall of a sleeve may be porous or semipermeable to allow entry of digestive secretions into the sleeve lumen and/or to allow the flow of fluids and digested matter out of the sleeve lumen.

In some embodiments, at least a portion of the wall of a sleeve may be impermeable, analogous to the Endobarrier(R) by GI Dynamics Inc, Watertown, Mass., USA and as described in U.S. Pat. No. 7,267,694.

The diameter of the sleeve lumen may be substantially constant along the entire length of the liner tube. Although any suitable luminal diameter may be used, in some embodiments, the luminal diameter may be not more than about 30 mm, not more than about 25 mm and even not more than about 20 mm.

In some embodiments, the proximal end of the sleeve may be flared and may define a funnel-like structure.

The length of the sleeve may be any suitable length and may be selected in accordance with clinical decisions made by the treating physician. A typical sleeve is between about 25 cm and about 160 cm long. Generally, the sleeve is selected so that when the duodenal sleeve device is deployed, the distal lumen opening of the sleeve is located distal to the duodenal-jejunal flexure and empties out into the jejunum. In some embodiments, the sleeve may be even longer.

Suitable materials from which the sleeve for implementing the invention are fashioned include silicone, polyurethane, polyethylene (e.g., low density polyethylene films) and fluoropolymers (e.g., expanded polytetrafluoroethylene). In some embodiments, the sleeve is fashioned from fluoropolymer or polyethylene film impregnated with polyurethane or silicone to reduce permeability, as taught in U.S. Pat. No. 7,267,694.

The sleeve may include one or more markers (e.g., barium) designed for viewing the position of the sleeve within the intestines through fluoroscopy, such as a longitudinal rib or other markers that are spaced along the length of sleeve. In addition, sleeve may further include components that inhibit twisting or kinking of the sleeve itself. In one embodiment, these components include one or more stiffening elements, such as rings, coupled to either the inside or the outside of the sleeve at spaced locations along its length. These rings can, for example, be made of a slightly thicker silicone material that would resist twisting or kinking of the sleeve around the ring. In other embodiments, the stiffening elements may be in spiral shape or extending lengthwise along at least a portion of the sleeve.

In a non-claimed implantation method, the sleeve may be initially folded or rolled up and packed into the interior of an applier. The distal end of sleeve may be initially closed, e.g. with a small polymeric or silicone seal and forms a programmed tearing line, e.g. a perforation, along which the distal end can tear open by the internal pressure of the chyme flow.

In this way bypass conduits can be created in the GI tract of a patient to achieve a malabsorptive effect in cases where such an effect may enhance weight loss, as well as the initially described effects on hormonal signaling in general.

Particularly, the described devices and procedures obviate undesired migration of the sleeve away from its original anchoring position and addresses the need of reliable sealing of the lined lumen. Moreover, some embodiments of the described devices and methods are beneficial with regard to permitting normal biological events, including vomiting, to occur.

Although preferred embodiments of the invention have been described in detail, it is not the intention of the applicant to limit the scope of the claims to such particular embodiments, but to cover all modifications and alternative constructions falling within the scope of the invention.

## Claims

1. Endoluminal sleeve device (1) for internally lining a section of the GI tract, comprising:
- a sleeve (2) configured for deployment inside the duodenum, the sleeve (2) having walls of a flexible material defining a sleeve lumen (3), a proximal end (4) defining a proximal lumen opening (5), and a distal end (6) defining a distal lumen opening (7),
- an anchoring portion (8) forming the proximal sleeve end (4), **characterized in that** the anchoring portion (8) comprising shape memory material having three separate phases corresponding to a first shape memory state, to a second shape memory state and to a third shape memory state, wherein:
A) in the first shape memory state the anchoring portion (8) is folded in a collapsed shape,
B) in the second shape memory state, the anchoring portion (8) is unfolded in an expanded tubular shape adapted to shape interfere with a target anchoring location in the GI Ttract,
C) in the third shape memory state, a stiffness of the anchoring portion (8) is reduced with respect to the stiffness of the anchoring portion (8) in the second shape memory state and the anchoring portion is foldable.

2. Endoluminal sleeve device (1) according to claim 1, in which in said third shape memory state one of a shape and a stiffness of the anchoring portion (8) is altered with respect to the shape and stiffness thereof in the second shape memory state and adapted for adjusting the anchoring portion to anatomy.

3. Endoluminal sleeve device (1) according to any one of the preceding claims, in which the anchoring portion (8) is configured that a transition between the shape memory states can be induced by a one of a temperature change, exposure to an electric field, exposure to a magnetic field or by light irradiation.

4. Endoluminal sleeve device (1) according to any one of the preceding claims, in which the shape memory material comprises a shape memory polymer.

5. Endoluminal sleeve device (1) according to any one of the preceding claims, in which the shape memory material comprises a combination of two different two-phase-shape memory polymers with different glass transition temperatures, thereby achieving three separate phases.

6. Endoluminal sleeve device (1) according to any one of the preceding claims, in which the anchoring portion (8) comprises an electrical heating resistance (9) with terminals for connecting an endoscopic electrode.

7. Endoluminal sleeve device (1) according to any one of claims 1 to 5, in which the anchoring portion (8) comprises an electrically conducting winding (11) with terminals for connecting an endoscopic electrode and adapted to generate an electromagnetic field in response to energizing.

8. Endoluminal sleeve device (1) according to any one of claims 1 to 5, in which the anchoring portion (8) comprises an electrical conductor (14) adapted to generate an induction electric current in response to a magnetic field variation.

9. Endoluminal sleeve device (1) according to any one of the preceding claims, in which protrusions (13) are formed on an external surface (15) of the anchoring portion (8), said protrusions (13) being covered by folds when the anchoring portion (8) is in the collapsed folded shape of the first shape memory state, and said protrusions (13) being exposed when the anchoring portion (8) is in the expanded unfolded shape of the second shape memory state.

10. Endoluminal sleeve device (1) according to any one of the preceding claims, in which the anchoring portion (8), when in the unfolded expanded shape, an external surface (15) of the anchoring portion (8) forms at least one cavity (12) into which surrounding tissue can invaginate for additional anchoring.

11. Endoluminal sleeve device (1) according to any one of the preceding claims, in which the anchoring portion (8) has a truncated cone shape in the second shape memory state.

12. Endoluminal sleeve device (1) according to any one of claims 1 to 10, in which the anchoring portion (8) has a circular cylindrical tube shape in the second shape memory state.

## Patentansprüche

1. Endoluminale Hülsenvorrichtung (1) zum internen Auskleiden eines Abschnitts des GI-Trakts, welche Folgendes umfasst:
- eine Hülse (2), die zum Einsatz innerhalb des Duodenums konfiguriert ist, wobei die Hülse (2) Wände aus einem flexiblen Material, die ein Hülsenlumen (3) definieren, ein proximales Ende (4), das eine proximale Lumenöffnung (5) definiert, und ein distales Ende (6), das eine distale Lumenöffnung (7) definiert, aufweist,
- einen Verankerungsbereich (8), der das proximale Hülsenende (4) bildet, **dadurch gekennzeichnet, dass** der Verankerungsbereich (8) ein Formgedächtnismaterial mit drei getrennten Phasen entsprechend einem ersten Formgedächtniszustand, einem zweiten Formgedächtniszustand und einem dritten Formgedächtniszustand umfasst, wobei:
A) der Verankerungsbereich (8) in dem ersten Formgedächtniszustand in eine zusammengelegte Form gefaltet ist,
B) der Verankerungsbereich (8) in dem zweiten Formgedächtniszustand in eine ausgedehnte röhrenförmige Form, die zur Formpresspassung mit einem Zielverankerungsort in dem GI-Trakt eingerichtet ist, ausgefaltet ist,
C) eine Steifigkeit des Verankerungsbereichs (8) in dem dritten Formgedächtniszustand mit Hinblick auf die Steifigkeit des Verankerungsbereichs (8) in dem zweiten Formgedächtniszustand reduziert ist und der Verankerungsbereich faltbar ist.

2. Endoluminale Hülsenvorrichtung (1) gemäß Anspruch 1, bei welcher eine Form oder eine Steifigkeit des Verankerungsbereichs (8) in dem dritten Formgedächtniszustand mit Hinblick auf die Form und die Steifigkeit davon in dem zweiten Formgedächtniszustand verändert ist, und zum Anpassen des Verankerungsbereichs an die Anatomie eingerichtet ist.

3. Endoluminale Hülsenvorrichtung (1) gemäß einem der vorhergehenden Ansprüche, bei welcher der Verankerungsbereich (8) derart konfiguriert ist, dass ein Übergang zwischen den Formgedächtniszuständen durch eine Temperaturänderung, eine Exposition gegenüber einem elektrischen Feld, eine Exposition gegenüber einem magnetischen Feld oder durch Lichtstrahlung induziert werden kann.

4. Endoluminale Hülsenvorrichtung (1) gemäß einem der vorhergehenden Ansprüche, bei welcher das Formgedächtnismaterial ein Formgedächtnispolymer umfasst.

5. Endoluminale Hülsenvorrichtung (1) gemäß einem der vorhergehenden Ansprüche, bei welcher das Formgedächtnismaterial eine Kombination von zwei verschiedenen Zwei-Phasen-Formgedächtnispolymeren mit unterschiedlichen Glasübergangstemperaturen umfasst, wodurch drei getrennte Phasen erreicht werden.

6. Endoluminale Hülsenvorrichtung (1) gemäß einem der vorhergehenden Ansprüche, bei welcher der Verankerungsbereich (8) einen elektrischen Heizwiderstand (9) mit Anschlüssen zum Verbinden einer Endoskopieelektrode umfasst.

7. Endoluminale Hülsenvorrichtung (1) gemäß einem der Ansprüche 1 bis 5, bei welcher der Verankerungsbereich (8) eine elektrisch leitende Windung (11) mit Anschlüssen zum Verbinden einer Endoskopieelektrode und eingerichtet zum Erzeugen eines elektromagnetischen Feldes in Reaktion auf eine Versorgung mit Energie umfasst.

8. Endoluminale Hülsenvorrichtung (1) gemäß einem der Ansprüche 1 bis 5, bei welcher der Verankerungsbereich (8) einen elektrischen Leiter (14) umfasst, der zum Erzeugen eines elektrischen Induktionsstroms in Reaktion auf eine Veränderung eines magnetischen Feldes eingerichtet ist.

9. Endoluminale Hülsenvorrichtung (1) gemäß einem der vorhergehenden Ansprüche, bei welcher Vorsprünge (13) auf einer äußeren Fläche (15) des Verankerungsbereichs (8) gebildet sind, wobei die Vorsprünge (13) von Falten bedeckt sind, wenn sich der Verankerungsbereich (8) in der zusammengelegten gefalteten Form des ersten Formgedächtniszustandes befindet, und wobei die Vorsprünge (13) freigelegt sind, wenn sich der Verankerungsbereich (8) in der ausgedehnten ungefalteten Form des zweiten Formgedächtniszustandes befindet.

10. Endoluminale Hülsenvorrichtung (1) gemäß einem der vorhergehenden Ansprüche, bei welcher, wenn sich der Verankerungsbereich (8) in der ungefalteten ausgedehnten Form befindet, eine äußere Fläche (15) des Verankerungsbereichs (8) zumindest einen Hohlraum (12) bildet, in welchen umgebendes Gewebe für ein zusätzliches Verankern eingestülpt werden kann.

11. Endoluminale Hülsenvorrichtung (1) gemäß einem der vorhergehenden Ansprüche, bei welcher der Verankerungsbereich (8) in dem zweiten Formgedächtniszustand eine Kegelstumpfform aufweist.

12. Endoluminale Hülsenvorrichtung (1) gemäß einem der Ansprüche 1 bis 10, bei welcher der Verankerungsbereich (8) in dem zweiten Formgedächtniszustand eine runde zylindrische Röhrenform aufweist.

## Revendications

1. Dispositif de manchon endoluminal (1) destiné au revêtement interne d'une section du tractus gastrointestinal, comprenant :
- un manchon (2) configuré pour être déployé à l'intérieur du duodénum, le manchon (2) ayant des parois en un matériau flexible définissant une lumière de manchon (3), une extrémité proximale (4) définissant une ouverture de lumière proximale (5), et une extrémité distale (6) définissant une ouverture de lumière distale (7),
- une partie d'ancrage (8) formant l'extrémité de manchon proximale (4), **caractérisée en ce que** la partie d'ancrage (8) comprenant un matériau à mémoire de forme a trois phases distinctes correspondant à un premier état de mémoire de forme, à un deuxième état de mémoire de forme, et à un troisième état de mémoire de forme, dans lequel :
A) dans le premier état de mémoire de forme, la partie d'ancrage (8) est pliée en une forme aplatie,
B) dans le second état de mémoire de forme, la partie d'ancrage (8) est dépliée en une forme tubulaire déployée adaptée à une interférence de forme avec un emplacement d'ancrage cible dans le tractus GASTRO-INTESTINAL,
C) dans le troisième état de mémoire de forme, une rigidité de la partie d'ancrage (8) est réduite relativement à la rigidité de la partie d'ancrage (8) dans le second état de mémoire de forme et la partie d'ancrage est pliable.

2. Dispositif de manchon endoluminal (1) selon la revendication 1, dans lequel dans ledit troisième état de mémoire de forme, l'une d'une forme et d'une rigidité de la partie d'ancrage (8) est modifiée relativement à la forme et à la rigidité de celle-ci dans le second état de mémoire de forme et adaptée à l'ajustement de la partie d'ancrage à l'anatomie.

3. Dispositif de manchon endoluminal (1) selon l'une quelconque des revendications précédentes, dans lequel la partie d'ancrage (8) est configurée de manière à ce qu'une transition entre les états de mémoire de forme puisse être induite par l'une d'une modification de la température, d'une exposition à un champ électrique, d'une exposition à un champ magnétique ou d'une irradiation de lumière.

4. Dispositif de manchon endoluminal (1) selon l'une quelconque des revendications précédentes, dans lequel le matériau à mémoire de forme comprend un polymère à mémoire de forme.

5. Dispositif de manchon endoluminal (1) selon l'une quelconque des revendications précédentes, dans lequel le matériau à mémoire de forme comprend une combinaison de deux différents polymères à mémoire de forme à deux phases avec des températures de transition vitreuse différentes, permettant ainsi d'obtenir trois phases distinctes.

6. Dispositif de manchon endoluminal (1) selon l'une quelconque des revendications précédentes, dans lequel la partie d'ancrage (8) comprend une résistance de chauffage électrique (9) avec des terminaux pour le raccordement à une électrode endoscopique.

7. Dispositif de manchon endoluminal (1) selon l'une des revendications 1 à 5, dans lequel la partie d'ancrage (8) comprend un enroulement électroconducteur (11) avec des terminaux pour le raccordement à une électrode endoscopique et adapté à générer un champ électromagnétique en réponse à la mise sous tension.

8. Dispositif de manchon endoluminal (1) selon l'une des revendications 1 à 5, dans lequel la partie d'ancrage (8) comprend un conducteur électrique (14) adapté à générer un courant électrique d'induction en réponse à une variation du champ magnétique.

9. Dispositif de manchon endoluminal (1) selon l'une quelconque des revendications précédentes, dans lequel des protubérances (13) sont formées sur une surface externe (15) de la partie d'ancrage (8), lesdites protubérances (13) étant couvertes par des plis lorsque la partie d'ancrage (8) se trouve dans la forme pliée et aplatie du premier état de mémoire de forme, et lesdites protubérances (13) étant exposées lorsque la partie d'ancrage (8) se trouve dans la forme dépliée et déployée du deuxième état de mémoire de forme.

10. Dispositif de manchon endoluminal (1) selon l'une quelconque des revendications précédentes, dans lequel la partie d'ancrage (8), lorsqu'elle se trouve sous une forme dépliée et déployée, a une surface externe (15) qui forme au moins une cavité (12) dans laquelle le tissu environnant peut s'invaginer pour un ancrage supplémentaire.

11. Dispositif de manchon endoluminal (1) selon l'une quelconque des revendications précédentes, dans lequel la partie d'ancrage (8) a une forme de cône tronqué dans le deuxième état de mémoire de forme.

12. Dispositif de manchon endoluminal (1) selon l'une des revendications 1 à 10, dans lequel la partie d'ancrage (8) a une forme de tube cylindrique circulaire dans le deuxième état de mémoire de forme.
